Europäisches Patentamt

European Patent Office

Office européen des brevets

(11) EP 0 733 338 A1

(19)

(12) DEMANDE DE BREVET EUROPEEN

(43) Date de publication:
25.09.1996 Bulletin 1996/39

(51) Int Cl.⁶: $A61B\ 3/113$, $G06F\ 3/00$

(21) Numéro de dépôt: 96400611.8

(22) Date de dépôt: 22.03.1996

(84) Etats contractants désignés:
DE GB IE

(30) Priorité: 24.03.1995 FR 9503489

(71) Demandeur: COMMISSARIAT A L'ENERGIE
ATOMIQUE
75015 Paris (FR)

(72) Inventeurs:
• Charbonnier, Colette
38800 Pont de Claix (FR)
• Masse, Dominique
38500 Coublevie (FR)

(74) Mandataire: Dubois-Chabert, Guy
c/o BREVATOME
25, rue de Ponthieu
75008 Paris (FR)

(54) Dispositif de mesure de la position du point de fixation d'un oeil sur une cible

(57) L'invention concerne un dispositif de mesure de la position du point de fixation (P') d'un oeil sur un écran vidéo (19) comportant :

• un support mobile (10) positionné à proximité de l'oeil et sur lequel sont fixés des premiers moyens d'éclairage (12) ainsi qu'une caméra vidéo (11) pour réaliser des images de l'oeil, et

• un support fixe (17) distant de l'oeil, et sur lequel sont positionnés l'écran vidéo orienté face à l'oeil, ainsi qu'une pluralité de sources lumineuses éclairant l'oeil en alternative avec les premiers moyens d'éclairage et disposées autour de l'écran vidéo pour créer sur l'oeil des reflets cornéens et des moyens de traitement (20) des images obtenues de l'oeil.

L'invention concerne aussi un procédé d'éclairage de l'oeil dans lequel l'oeil est éclairé par des flashes émis chacun en fin de chaque prise d'images ainsi qu'une application du dispositif à l'affichage, sur l'écran vidéo, d'images qui changent selon les mouvements de l'oeil, dans laquelle une zone de haute résolution (Z) est définie autour du point de fixation (P') et à déplacer cette zone selon les mouvements de l'oeil.

Application au domaine médical, à l'ergonomie et à l'informatique interactive.

FIG. 2

## Description

<u>Domaine de l'invention</u>

L'invention concerne un dispositif pour mesurer le positionnement du point de fixation d'un oeil sur une cible telle qu'un écran vidéo. Elle concerne aussi un procédé d'éclairage de l'oeil mis en oeuvre par ce dispositif, ainsi qu'une application à l'affichage d'images selon le regard du sujet et, plus précisément, d'affichage d'une zone d'intérêt autour du point de fixation de l'oeil et de déplacement de cette zone d'intérêt en fonction du regard du sujet.

Elle trouve des applications dans le domaine de l'informatique interactive où les informations présentées à l'écrans sont fonction de la position du regard. Plus particulièrement, elle s'applique au domaine de l'ergonomie pour l'analyse de la stratégie de prise d'information visuelle et de la fatigue d'un sujet travaillant sur un poste de travail. Elle s'applique aussi au domaine médical pour l'aide aux handicapés tétraplégiques qui peuvent activer des commandes en désignant des cases fonctionnelles sur un écran.

<u>Etat de la technique</u>

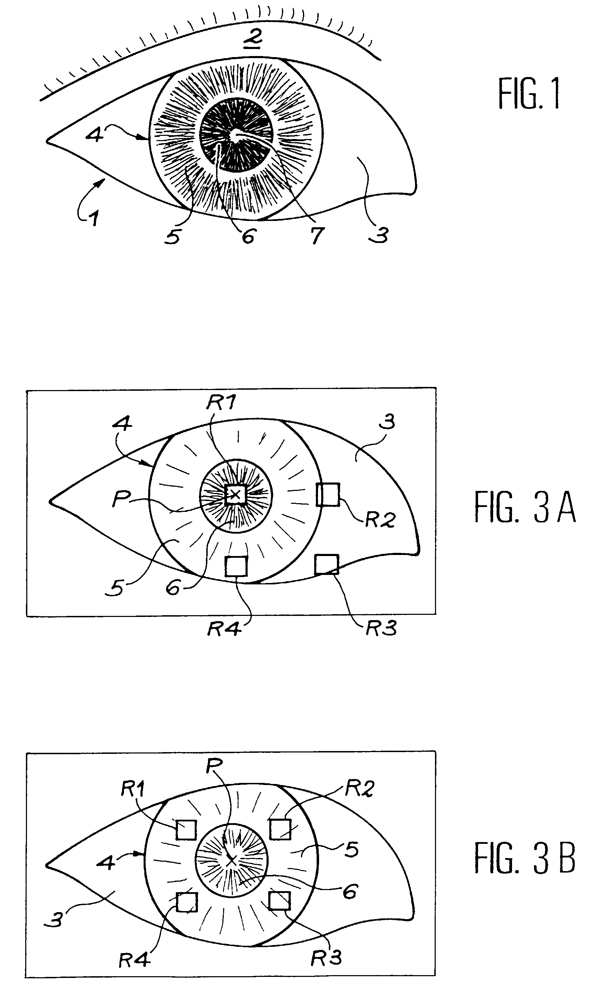

L'oeil est connu pour être un organe de l'homme qui suscite de nombreuses études, notamment sur ses mouvements. On a représenté sur la figure 1, schématiquement, un oeil vu de face. Cet oeil comporte une orbite 1 au-dessus de laquelle se trouve la paupière 2. Dans l'orbite 1, on peut voir une partie de la sclérotique 3 qui forme une sorte de sphère blanche, présentant une ouverture circulaire où est insérée la cornée 4, un peu plus bombée que la sclérotique 3. La cornée 4 est transparente et permet de discerner, par transparence, l'iris 5 qui est une membrane opaque percée d'une ouverture circulaire, à savoir la pupille 6. sur cette figure, on a représenté, également, un reflet cornéen 7, c'est-à-dire le reflet que l'on peut distinguer sur la cornée 4 lorsque l'oeil est éclairé de face.

Comme on le verra dans la suite de la description, plusieurs reflets cornéens peuvent être visibles sur la pupille 6, la sclérotique 3 ou sur l'iris 5 lorsque plusieurs sources lumineuses éclairent l'oeil.

Habituellement, la mesure du mouvement d'un oeil consiste à déterminer la position de la pupille et d'un reflet cornéen de l'oeil. Cependant, lorsque l'oeil est éclairé sans précautions particulières, les images réalisées peuvent contenir certaines parties sombres, comme les cils et les sourcils, qui se confondent avec le disque sombre de la pupille. De même, certaines parties claires de l'image réalisée, telles que les reflets sur la peau des paupières 2 ou le blanc de la sclérotique 3, peuvent être confondues avec la tâche brillante du reflet cornéen 7. Ces parties respectivement sombres ou claires faussent le calcul du centre, respectivement, de la pupille ou du reflet cornéen.

la demande de brevet FR-A-2 684 285 propose un procédé et un dispositif permettant d'améliorer la qualité des images à traiter et donc la précision des mesures de position de l'oeil déterminée à partir de ces images. Ce procédé consiste à éclairer l'oeil, alternativement ou simultanément, par deux sources lumineuses de directions distinctes.

Selon ce document, les mouvements de l'oeil sont mesurés dans un repère lié à la tête du sujet. Il nécessite donc une certaine contrainte pour le sujet. En effet, pour connaître la position du regard sur un écran, il est donc nécessaire d'immobiliser la tête du sujet devant l'écran.

En outre, ce procédé et de dispositif n'ont pas pour objectif la détermination de la position du point de regard sur un écran vidéo, en temps réel.

Il existe toutefois des systèmes de mesure permettant de déterminer la position du point de fixation d'un oeil sur un écran vidéo. Un de ces systèmes est décrit, par exemple, dans l'article "A study of human interface using an eye-movement detection system" de M. IIDA, A. TOMONO et Y. KOBAYASHI dans ATR Communication Systems Research Laboratories, Sanpeidani, Inuidani, Seika-cho, Souraku-gun, Kyoto 619-02 (Japan). Un tel système présente l'inconvénient de nécessiter une immobilisation totale ou partielle de la tête du patient devant l'écran vidéo ou bien de nécessiter l'ajout d'un système de mesure des mouvements de la tête du sujet.

<u>Exposé de l'inventon</u>

L'invention a justement pour but de remédier aux inconvénients des procédés et dispositifs décrits précédemment et de permettre la mise en oeuvre de systèmes interactifs dans lesquels les informations présentées à l'écran sont fonction de la position du regard sur l'écran et ne nécessitent ni contrainte de la tête du sujet, ni mesure des mouvements de la tête du sujet. A cette fin, elle propose un dispositif et un procédé d'éclairage de l'oeil pour mesurer la position du point de fixation du regard sur un écran vidéo et un procédé pour afficher des images en fonction du mouvement de l'oeil.

De façon plus précise, l'invention concerne un dispositif de mesure de la position du point de fixation d'un oeil sur une cible, comportant un support mobile solidaire de la tête du sujet, positionné à proximité de l'oeil et sur lequel sont fixés des premiers moyens d'éclairage de l'oeil ainsi que des moyens vidéo pour réaliser des images de l'oeil. Ce dispositif comporte, en outre, un support fixe distant de l'oeil et sur lequel sont positionnés, d'une part, la cible orientée de façon à être sensiblement face à l'oeil et, d'autre part, des seconds moyens d'éclairage de l'oeil aptes à créer sur l'oeil des reflets cornéens.

Selon l'invention, la cible consiste en des moyens d'affichage d'images tels qu'un écran vidéo.

Avantageusement, les seconds moyens d'éclairage de l'oeil comportent une pluralité de sources de lumière infrarouge disposées autour des moyens d'affichage d'images, chaque source de lumière créant sur l'oeil un reflet cornéen.

Selon l'invention, les premiers moyens d'éclairage et les seconds moyens d'éclairage illuminent l'oeil séquentiellement et les moyens vidéo réalisent une image de l'oeil à chacun des éclairages.

Le dispositif comporte de plus des moyens de traitement d'images aptes :

- à déterminer, d'une part, le centre de gravité de la pupille de l'oeil à partir d'une image obtenue lorsque l'oeil est éclairé par les premiers moyens d'éclairage et, d'autre part, le positionnement de chaque reflet cornéen par rapport au centre de la pupille à partir d'une image obtenue lorsque l'oeil est éclairé par les seconds moyens d'éclairage, et
- à déterminer la position du point de fixation sur les moyens d'affichage d'images à partir de la mesure de la position du centre de la pupille sur l'oeil.

L'invention concerne aussi un procédé d'éclairage de l'oeil destiné à réduire l'écart temporel entre la position de l'oeil et la nature correspondante. Ce procédé consiste à éclairer l'oeil par des flashes émis chacun en fin de chaque prise d'image de l'oeil.

L'invention concerne de plus une application du dispositif à l'affichage d'images utilisant le procédé d'éclairage ci-dessus. Cette application consiste à déterminer, sur une image affichée sur les moyens d'affichage, une zone d'intérêt (Z) située autour du point de fixation (P') et à déplacer cette zone d'intérêt en fonction des mouvements de l'oeil.

L'oeil effectuant des saccades, le déplacement de la zone d'observation est effectué entre un début et une fin de saccade.

Brève description des figures

- la figure 1, déjà décrite, représente une vue de face d'un oeil,
- la figure 2 représente, schématiquement, le dispositif de mesure de la position du point de fixation conforme à l'invention,
- les figures 3A et 3B représentent chacune, de façon schématique, un exemple d'oeil sur lequel apparaissent des reflets cornéens ainsi que la position du centre de gravité de la pupille,
- la figure 4A représente un exemple d'oeil avec des reflets cornéens et le centre de gravité de la pupille,
- la figure 4B représente le repère dans lequel sont calculées les positions des reflets cornéens montrés sur l'oeil de la figure 4A par rapport à la position du centre de gravité de la pupille,
- la figure 4C représente l'écran vidéo sur lequel est noté le repère homothétique à celui de la figure 4B,
- la figure 5 montre des courbes temporelles représentatives d'une saccade de l'oeil réelle et de saccades de l'oeil mesurées ; et
- la figure 6 représente un diagramme temporel expliquant l'éclairage de l'oeil pendant la prise d'images.

Description de modes de réalisation détaillés de l'invention

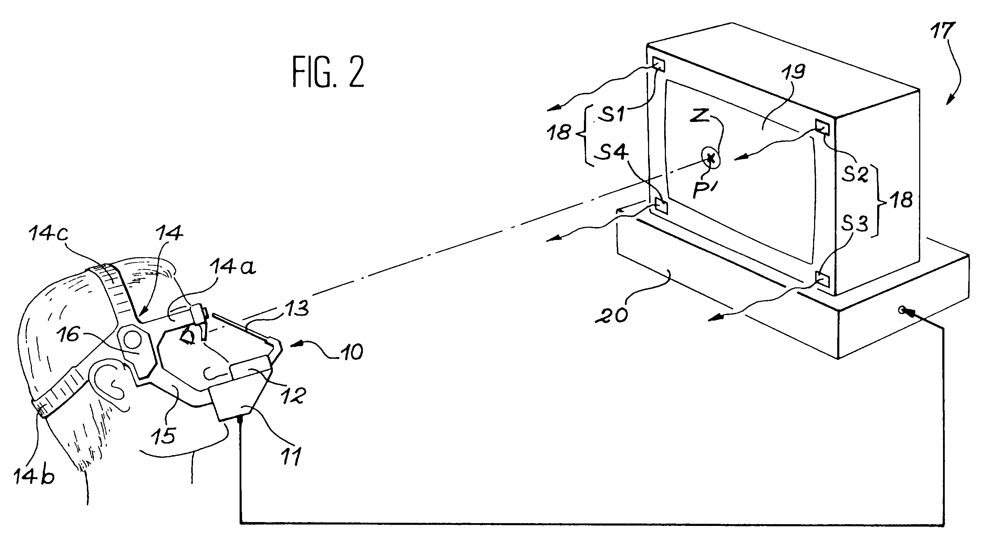

Sur la figure 2, on a représenté schématiquement le dispositif de l'invention destiné à mesurer la position du point de fixation de l'oeil sur l'écran vidéo.

Ce dispositif comprend un ensemble 10 de mesure mobile, ou support mobile, pouvant être disposé à proximité de l'oeil. Ce support mobile 10 comporte des moyens de prise d'images tels qu'une caméra vidéo CCD miniature 11, et une source lumineuse 12 qui émet des rayons lumineux vers le miroir semi-transparent 13 incliné de façon à renvoyer la lumière sur l'oeil du sujet dont on cherche à déterminer la position du regard sur la cible 19.

Ce support mobile 10 peut être installé sur une monture de lunette, un casque ou tout autre support adaptable sur la tête du sujet.

Selon l'exemple de réalisation montré sur cette figure 2, l'ensemble 10 de mesure est monté sur une monture de lunette 14. La caméra 11 est fixée sur la monture de lunette 14 par un support de caméra 15. Des moyens 16 de réglage mécaniques permettent de régler la position du support de caméra 15 par rapport à la monture 14 afin de rendre le dispositif adaptable à tout sujet. Ces moyens de réglage 16 sont situés sur la monture de lunette 14, plus précisément sur la branche 14a de la monture, les deux branches pouvant être reliées par une ou plusieurs bandes de liaison 14b, 14c, souples, du type sangles, permettant de s'affranchir de la plupart des mouvements résiduels de la monture de lunette 14 par rapport à la tête du sujet.

Le dispositif de l'invention comprend en outre un support fixe 17 sur lequel sont positionnés des moyens d'affichage d'images 19, ou écran vidéo, constituant la cible regardée par l'oeil du sujet. Sur ce support fixe 17, sont également fixés des seconds moyens d'éclairage 18, les premiers moyens d'éclairage étant la source 12 montée sur le support mobile. Ces moyens d'éclairage 18 comportent une pluralité de sources de lumière infrarouge disposées autour de l'écran vidéo 19. Sur le mode de réalisation représenté sur la figure 2, ces sources sont au nombre de quatre réparties à chaque coin de l'écran vidéo 19. Elles sont référencées S1, S2, S3 et S4. Ces sources S1, S2, S3 et S4, lorsqu'elles illuminent l'oeil, créent sur la pupille, l'iris ou même la sclérotique de l'oeil des reflets, respectivement R1, R2, R3 et R4, nommés reflets cornéens.

Le dispositif de l'invention comporte de plus des moyens de traitement 20 destinés à déterminer, à partir des images prises de l'oeil, la position du point de fixation sur la cible 19, c'est-à-dire sur l'écran vidéo 19.

La caméra 11 est connectée électriquement aux moyens de traitements 20 de façon à ce que les images de l'oeil obtenues par la caméra soient envoyées directement aux moyens de traitement 20 pour être traitées. Ces moyens de traitement 20 permettent de déterminer, d'une part, le centre de gravité de la pupille et, d'autre part, la position des reflets cornéens par rapport à ce centre de pupille. Le procédé de calcul de ces données sera décrit de façon plus détaillée dans la suite de la description.

Ces moyens de traitement sont positionnés de préférence sur le support fixe 17. Selon un mode de réalisation de l'invention, ces moyens de traitement 20 associés à l'écran vidéo 19 constituent un ordinateur.

Sur l'écran vidéo 19, on a représenté, par une croix, la position P' du point de fixation de l'oeil du sujet sur l'écran et par un carré, la zone d'intérêt Z entourant ce point de fixation. Cette zone d'intérêt est une zone d'observation qui correspond à une portion du champ visuel couvert par la fovéa de l'oeil. Elle correspond à une zone centrée autour du point P' et pour laquelle l'acuité du sujet est la meilleure, par rapport à son acuité pour l'ensemble du champ de vision. Son rôle sera décrit de façon plus détaillée dans la suite de la description.

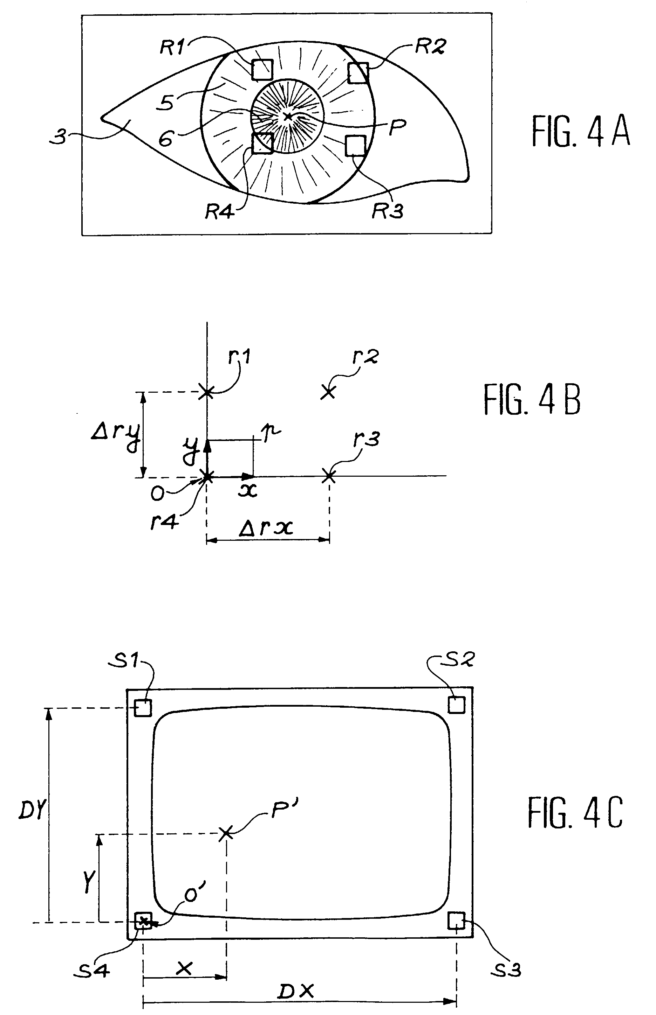

Sur les figures 3A et 3B, on a représenté schématiquement deux exemples de reflets cornéens sur un oeil.

Comme expliqué précédemment, les reflets cornéens sont les reflets des sources de lumière S1 à S4 sur la cornée 4 ou la sclérotique 3 de l'oeil. La position de ces reflets sur l'oeil dépend de la position du regard par rapport à l'écran vidéo et donc par rapport aux sources lumineuses S1 et S4 qui entourent l'écran vidéo.

La figure 3A illustre un exemple de reflets cornéens R1, R2, R3 et R4 sur l'oeil. Le point P représente la position du centre de gravité de la pupille 6. Ce centre de gravité P de la pupille 6 est détecté et calculé à partir d'une image réalisée par la caméra lorsque l'oeil est éclairé par la source lumineuse 12 située sur le support mobile. Les reflets cornéens R1 à R4 sont détectés à partir d'une image réalisée par la caméra lorsque l'oeil est éclairé par les sources lumineuses S1 à S4 situées sur le support fixe. Ces deux moyens d'éclairage (d'une part la source 12, d'autre part les sources S1 à S4) illuminent donc séquentiellement l'oeil et, à chaque séquence d'éclairage, une image de l'oeil est prise. Une paire d'images de l'oeil est donc nécessaire pour déterminer la position des reflets R1 et R4 par rapport au centre P de la pupille 6.

La figure 1 montre une image du type pupille obtenue avec les premiers moyens d'éclairage. Les figures 3A, 3B montrent une image du type reflet obtenue avec les seconds moyens d'éclairage d'un oeil.

Sur l'exemple de la figure 3A, le reflet R1 est confondu avec le centre P de la pupille ; les reflets R2 et R3 sont positionnés à cheval sur l'iris 5 et la sclérotique 3 de l'oeil ; et le reflet R4 est situé sur l'iris 5 de l'oeil. Sachant que le centre P de la pupille apparaît sur le reflet de l'objet regardé, on en déduit que, dans le cas de la figure 3A, l'oeil regarde la source lumineuse S1 qui produit sur l'oeil le reflet R1.

La figure 3B illustre un autre exemple de reflets cornéens sur un oeil. Selon cet exemple, le centre P de la pupille 6 constitue sensiblement le centre du polygone formé par les reflets R1 à R4. Plus précisément, les reflets R1, R2, R3 et R4 sont situés sur l'iris 5, symétriquement les uns aux autres par rapport au centre P de la pupille. On en déduit qu'au moment de la prise d'images, le sujet était en train de regarder un point voisin du centre de l'écran vidéo.

Les figures 4A, 4B et 4C montrent de façon plus précise comment est déterminée la position du point de fixation P' sur l'écran à partir des positions des reflets cornéens R1 à R4 par rapport au centre P de la pupille.

La figure 4A montre encore un exemple de reflets cornéens créés sur un oeil par les sources lumineuses S1, S2, S3 et S4. Dans cet exemple, les reflets cornéens R1 et R2 sont positionnés sur l'iris 5 de l'oeil ; le reflet R3 est situé en limite de la sclérotique 3 ; le reflet R4 est situé à cheval sur l'iris 5 et la pupille 6.

Sur la figure 4B, on a représenté le repère de l'oeil à partir duquel sont déterminées les positions des reflets R1 à R4 par rapport au centre P de la pupille. Si l'on considère, comme exemple, l'oeil montré sur la figure 4A, le reflet R4 a pour centre de gravité r4 qui constitue l'origine O du repère. Le centre de gravité r3 du reflet R3 est sur l'axe des abscisses du repère, à une distance $\Delta Rx$ de l'origine O. Le centre de gravité r1 du reflet R1 est sur l'axe des ordonnées du repère à une distance $\Delta Ry$ de l'origine O ; et le centre de gravité r2 du reflet R2 est situé à une distance $\Delta Rx$ sur l'axe des abscisses et $\Delta Ry$ sur l'axe des ordonnées par rapport à l'origine O.

Le centre p de la pupille est situé, en abscisse, à une distance x de l'origine et, en ordonnée, à une distance y de l'origine O, c'est-à-dire du centre de gravité r4.

Les coordonnées (x, y) constituent ainsi la position du centre p de la pupille par rapport aux centres de gravité r1, r2, r3 et r4 des reflets respectifs R1, R2, R3 et R4.

Sur la figure 4C, on a représenté le repère lié à l'écran vidéo. La source lumineuse S4 constitue l'origine O' de ce repère. Les distances Dx et Dy sont, respectivement, les distances en abscisse et en ordonnée des sources S3 et S1 par rapport à l'origine O'. La source S2 est à une distance Dx en abscisse et à une distance Dy en ordonnée de l'origine O'.

En considérant que le repère lié à l'écran (O', X, Y) est une homothétie du repère lié à l'oeil (O, x, y), on peut déterminer la position du point de fixation P' de l'oeil sur l'écran grâce à la transformation homothétique suivante :

$$\left\{ \begin{array}{ll} E1 : & \dfrac{x}{\Delta rx} = \dfrac{X}{DX} \\[4mm] E2 : & \dfrac{y}{\Delta ry} = \dfrac{Y}{DY} \end{array} \right.$$

ce qui conduit aux coordonnées (X, Y) du point de fixation P' sur l'écran :

$$P' : \left\{ \begin{array}{l} X = x\,\dfrac{DX}{\Delta rx} \\[6mm] Y = y\,\dfrac{DY}{\Delta ry} \end{array} \right.$$

On comprend ainsi que les coordonnées (x, y) du centre P de la pupille sur l'oeil correspondent, à une homothétie près, à la position du regard de sujet sur l'écran.

Dans la pratique, il est souvent préférable d'appliquer aux expressions E1 et E2 des coefficients correctifs qui tiennent compte des imperfections géométriques de la cornée et des distorsions de l'image des sources S1 à S4 sur la cornée.

Ce dispositif peut permettre, par exemple, de réaliser une "souris oculaire" pour remplacer la souris bien connue, qui est connectée à l'ordinateur. Le sujet peut alors sélectionner ses choix sur des menus affichés simplement par son regard.

Le dispositif de mesure de la position du point de fixation P' sur un écran décrit précédemment peut aussi être utilisé au sein d'un système de visualisation contrôlé par la direction du regard. Dans ce cas, il est nécessaire d'effectuer des mesures en temps réel.

Cependant, il existe un certain écart temporel entre la mesure obtenue et la position réelle de l'oeil. L'invention propose donc un procédé permettant de réduire cet écart temporel.

Une des applications est l'affichage sur un écran d'une image à résolution non uniforme.

Pour afficher une telle image, le procédé de l'invention tient compte d'une caractéristique du système visuel de l'homme. En effet, l'oeil ne saisit pas tout son champ de vision avec la même acuité : seule une zone centrée autour du point de fixation de l'oeil est vue avec la meilleure définition, le reste du champ de vision étant vu avec une définition réduite. Le procédé consiste donc à utiliser cette caractéristique naturelle de l'oeil en définissant autour du point de fixation sur l'écran, une zone dite "d'intérêt". Cette zone d'observation est également nommée zone de haute résolution, par contraste avec le restant de l'image qui a une faible résolution. Cette zone d'observation est représentée sur la figure 2 par le cercle Z entourant le point P' de fixation de l'oeil sur l'écran.

Le procédé consiste ensuite à asservir cette zone d'intérêt autour du point de fixation quels que soient les mouvements de l'oeil. En d'autres termes, le procédé de l'invention a pour but d'assurer le déplacement en temps quasi réel, de la zone d'observation Z définie autour du point de fixation P', en fonction des mouvements de l'oeil.

Ce procédé est donc mis en oeuvre par le dispositif de mesure décrit précédemment puisqu'il utilise la mesure de position déterminée par ce dispositif.

Le procédé de l'invention tient compte aussi d'une seconde caractéristique de l'oeil humain. En effet, lors de l'inspection du champ visuel, la vision est interrompue plusieurs fois par seconde par des saccades, c'est-à-dire les mouvements rapides de l'oeil d'un point de fixation à un autre. Ces mouvements peuvent avoir une durée variant de 20 à 60 ms selon leur amplitude.

Dans le cas d'un système de présentation d'images évoluant à l'insu du sujet mais en fonction de son regard, il est nécessaire d'afficher les images, c'est-à-dire de déplacer la zone d'observation Z suffisamment rapidement pour que le sujet ne s'aperçoive pas que seule une zone de l'image a une haute résolution, le restant de l'image étant de faible résolution.

L'invention propose donc, pour permettre ces modifications de stimulation visuelle, d'effectuer le changement d'image affichée, c'est-à-dire de déplacer la zone d'observation, pendant une saccade ou juste à la fin de cette saccade.

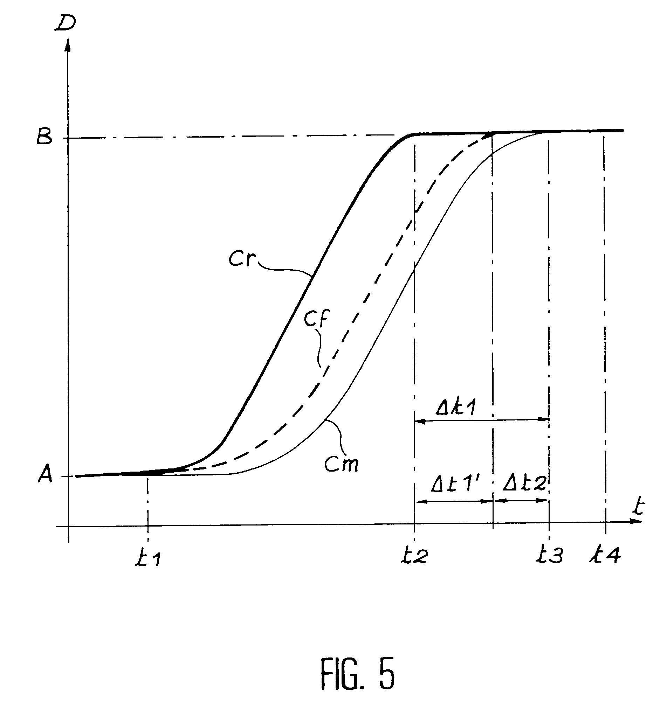

On a représenté sur la figure 5 une courbe des temps pour une saccade réelle (Cr) et une courbe des temps pour une saccade mesurée (Cm). La courbe Cr de cette figure 5 montre le déplacement, sur l'axe D, de l'oeil en fonction du temps t. Selon l'exemple représenté par cette courbe Cr, l'oeil bouge de façon à passer du point de fixation A au point de fixation B entre les instants t1 et t2, t1 correspondant au début de la saccade et t2 à la fin de la saccade. Pour modifier la stimulation visuelle au point de fixation B sans que le sujet s'en aperçoive, il faut effectuer le changement d'affichage entre les instants tl et t4, t4 étant un instant situé peu après t2, c'est-à-dire un peu après l'arrivée de l'oeil à la position B. A cet instant t4, l'oeil est arrivé à son nouveau point de fixation B depuis t4-t2. Cet écart temporel entre la fin de saccade t2 et l'instant t3 est nommé $\Delta t0$ ; cet écart peut varier selon l'individu et la nature de la stimulation visuelle ; en moyenne, elle équivaut à environ 10 ms.

Pour le procédé de l'invention, on choisit d'effectuer la modification de la stimulation visuelle lorsque l'oeil a atteint son point de fixation B, c'est-à-dire entre les instants t2 et t4. Le temps demandé pour le refroidissement de l'écran et le calcul de l'image ajoute au retard $\Delta t1$ dû au système de mesure du point de fixation P', un retard $\Delta t2$. Le changement d'affichage intervient donc $\Delta t1+\Delta t2$ après la fin de la saccade réelle.

La courbe Cm représente la courbe temporelle de la saccade mesurée. L'instant t3 de la fin de la saccade mesurée est décalé de $\Delta t1$ par rapport à la fin de la saccade réelle.

Afin de diminuer $\Delta t1$, l'invention propose d'éclairer l'oeil au moyen de flash. Cet éclairage par flash a également pour avantage que, lorsque l'oeil effectue une saccade, il se déplace pendant le temps de la prise d'image, c'est-à-dire pendant la durée d'une trame de la caméra. Pour une meilleure compréhension, on précise que pour fournir une image, une caméra vidéo de type CCD intègre la lumière reçue pendant la durée d'une trame, soit de l'ordre de 16ms pour une caméra fournissant 60 images/seconde.

Si pendant la durée d'une trame, une saccade survient au cours de laquelle l'oeil se déplace, alors l'image obtenue est floue et la mesure de la direction du regard est erronée. Le procédé de l'invention propose donc de réaliser des images instantanées de l'oeil en éclairant cet oeil par des flashes lumineux. Ces flashes peuvent être émis à n'importe quel moment de la prise d'image. Cette prise d'image correspond à l'intégration de la lumière par les détecteurs CCD de la caméra, pendant la durée d'une trame incluant un flash.

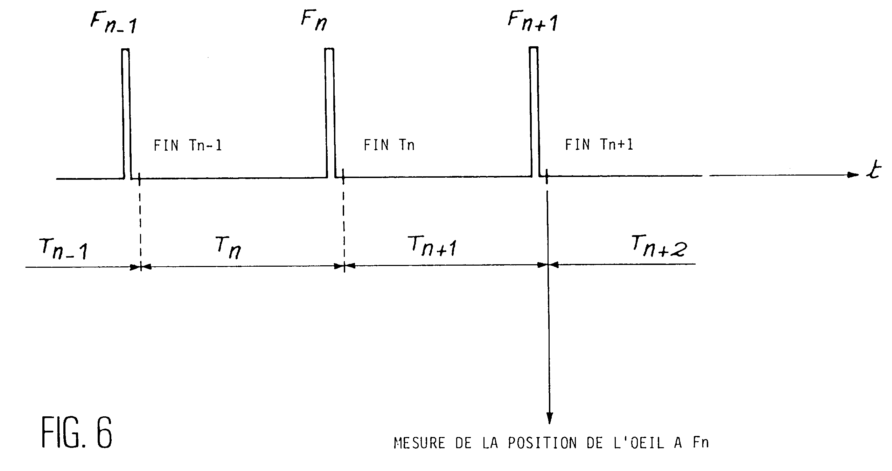

De plus, en éclairant l'oeil par un flash en fin de chaque trame, on peut limiter l'écart temporel entre la mesure de la position de l'oeil à l'instant du flash et l'instant du flash lui-même, à la durée d'une trame. On a représenté sur la figure 6, un diagramme temporel indiquant la position temporelle des flashes Fn-1, Fn et Fn+1 pendant les trames Tn-1 à Tn+2. Cette figure 6 montre que chaque flash Fn-1, Fn et Fn+1 intervient juste avant la fin de sa trame correspondante, respectivement Tn-1, Tn et Tn+1. L'instant de chaque fin de trame Tn-1, Tn, Tn+1 est indiqué sur l'axe des temps t par FIN Tn-1, FIN Tn et FIN Tn+1.

Considérons l'exemple de la prise d'image de l'oeil pendant la trame Tn : à la fin d'une première trame Tn, le flash Fn est déclenché pour illuminer l'oeil ; la lecture des détecteurs CCD par l'électronique de la caméra CCD se fait pendant la trame suivante Tn+1. A la fin de la trame suivante Tn+1, après lecture des détecteurs CCD de la caméra, la mesure de la position de l'oeil à l'instant du flash Fn est déterminée et simultanément l'oeil est éclairé par le flash suivant Fn+1, juste avant la fin de la trame Tn+1 (l'instant où cette mesure est effectuée est également représenté sur la figure 6 pour une meilleure compréhension du procédé d'éclairage).

Cette figure 6 permet donc de comprendre comment le procédé d'éclairage de l'oeil de l'invention permet de réduire le retard entre l'instant d'obtention de la mesure de position de l'oeil et l'instant d'éclairage.

La courbe Cf sur la figure 5, représente la courbe temporelle de la saccade mesurée lorsqu'il y a éclairage par flash. Elle montre qu'en éclairant l'oeil de la façon expliquée précédemment, la saccade mesurée Cf n'est décalée que d'un intervalle $\Delta t1'$ (avec $\Delta t1' < \Delta t1$) de la saccade réelle Cr. Cet éclairage par flash a donc le double avantage d'assurer une image de l'oeil nette et de réduire le retard entre la prise d'image et le résultat de la mesure pour permettre un affichage d'images quasiment en temps réel.

## Revendications

1. Dispositif de mesure de la position du point de fixation (P') d'un oeil sur une cible, comportant un support mobile (10) solidaire de la tête du sujet, positionné à proximité de l'oeil et sur lequel sont fixés des premiers moyens d'éclairage de l'oeil (12) ainsi que des moyens vidéo (11) pour réaliser des images de l'oeil, caractérisé en ce qu'il comporte un support fixe (17) distant de l'oeil et sur lequel sont positionnés, d'une part, la cible (19) orientée de façon à être sensiblement face à l'oeil et, d'autre part, des seconds moyens d'éclairage de l'oeil (18) aptes à créer, sur l'oeil, des reflets cornéens (R1, R2, R3, R4).

2. Dispositif de mesure selon la revendication 1, caractérisé en ce que la cible consiste en des moyens d'affichage d'images.

3. Dispositif de mesure selon la revendication 2, caractérisé en ce que les seconds moyens d'éclairage de l'oeil comportent une pluralité de sources de lumière infrarouge (S1, S2, S3, S4) disposées autour des moyens d'affichage d'images, chaque source de lumière créant sur l'oeil un reflet cornéen.

4. Dispositif de mesure selon l'une quelconque des revendications 1 à 3, caractérisé en ce que les premiers moyens d'éclairage et les seconds moyens d'éclairage illuminent l'oeil séquentiellement et les moyens vidéo réalisent une image de l'oeil pour chacun des éclairages.

5. Dispositif de mesure selon la revendication 4, caractérisé en ce qu'il comporte des moyens de traitement d'images (20) aptes, d'une part, à déterminer le centre de gravité de la pupille de l'oeil à partir d'une image obtenue lorsque l'oeil est éclairé par les premiers moyens d'éclairage ainsi que le positionnement de chaque reflet cornéen par rapport au centre de la pupille à partir d'une image obtenue lorsque l'oeil est éclairé par les seconds moyens d'éclairage et, d'autre part, à déterminer la position du point de fixation (P') sur les moyens d'affichage d'images à partir de la mesure de la position du centre de la pupille sur l'oeil.

6. Procédé d'éclairage d'un oeil destiné à effectuer des prises d'images de cet oeil, mis en oeuvre par le dispositif conforme à l'une quelconque des revendications 1 à 5, caractérisé en ce qu'il consiste à éclairer l'oeil par des flashes émis chacun en fin de chaque prise d'images de l'oeil.

7.  Application du dispositif selon l'une quelconque des revendications 1 à 5 à l'affichage d'images dont les images affichées changent en fonction des mouvements de l'oeil et dans laquelle l'oeil est éclairé conformément au procédé selon la revendication 6, caractérisée en ce qu'elle consiste à déterminer, sur une image affichée sur les moyens d'affichage, une zone d'intérêt (Z) située autour du point de fixation (P') et à déplacer cette zone d'intérêt en fonction des mouvements de l'oeil.

8.  Application selon la revendication 7, caractérisée en ce que, l'oeil effectuant des saccades, le déplacement de la zone d'intérêt est effectué pendant un court intervalle de temps (t2, t4) après la fin de la saccade.

FIG. 1

FIG. 3 A

FIG. 3 B

FIG. 2

EP 0 733 338 A1

FIG. 4 A

FIG. 4 B

FIG. 4 C

FIG. 5

EP 0 733 338 A1

$F_{n-1}$       $F_n$       $F_{n+1}$

FIN Tn-1     FIN Tn     FIN Tn+1

$t$

$T_{n-1}$     $T_n$     $T_{n+1}$     $T_{n+2}$

FIG. 6

MESURE DE LA POSITION DE L'OEIL A Fn

# EP 0 733 338 A1

Office européen
des brevets

# RAPPORT DE RECHERCHE EUROPEENNE

Numero de la demande

EP 96 40 0611

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X<br>Y | WO-A-92 21999 (HUMPHREY INSTRUMENTS INC.)<br>* page 15, ligne 3 - page 16, ligne 2 *<br>* page 18, ligne 8 - ligne 18; figures 1-11 * | 1,3<br>2,4 | A61B3/113<br>G06F3/00 |
| D,Y<br>A | EP-A-0 547 931 (COMMISSARIAT A L'ENERGIE)<br>* le document en entier * | 2<br>7 | |
| Y<br>A | EP-A-0 588 290 (NIKON CORPORATION)<br>* colonne 4, ligne 11 - colonne 5, ligne 54; figures 1-3,9,10,16 * | 4<br>5 | |
| A | US-A-4 852 988 (J.VELEZ ET AL.)<br>* le document en entier * | 1-3 | |
| A | GB-A-2 276 467 (UNIVERSITY COLLEGE LONDON)<br>* page 7, ligne 13 - ligne 36; figures 1-3 * | 1,3,4 | |
| A | DATABASE INSPEC<br>INSTITUTE OF ELECTRICAL ENGINEERS,<br>STEVENAGE, GB<br>Inspec No. 5064677,<br>CHARBONNIER C ET AL: "Gaze-controlled display"<br>XP002006353<br>* abrégé *<br>& ORIA 94. FROM TELEPRESENCE TOWARDS VIRTUAL REALITY, PROCEEDINGS ORIA 94. 5TH INTERNATIONAL SYMPOSIUM AND BUSINESS CONVENTION, MARSEILLE, FRANCE, 5-6 DEC. 1994, 1994, MARSEILLE, FRANCE, INST. INT. ROBOTIQUE & INTELLIGENCE ARTIFICIELLE MARSEILLE, FRANCE,<br>pages 291-296, | 1,6-8 | **DOMAINES TECHNIQUES RECHERCHES (Int.Cl.6)**<br>A61B<br>G06F |

-/--

Le présent rapport a été établi pour toutes les revendications

| Lieu de la recherche | Date d'achèvement de la recherche | Examinateur |
|---|---|---|
| LA HAYE | 24 Juin 1996 | Hunt, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

13

Office européen
des brevets

**RAPPORT DE RECHERCHE EUROPEENNE**

Numero de la demande

EP 96 40 0611

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int.Cl.6) |
| A | US-A-5 325 133 (T.ADACHI)<br>* colonne 3, ligne 33 - colonne 4, ligne 32; figures 1,2 *<br>----- | 1 | |
| | | | DOMAINES TECHNIQUES RECHERCHES (Int.Cl.6) |
| Le présent rapport a été établi pour toutes les revendications | | | |

| Lieu de la recherche | Date d'achèvement de la recherche | Examinateur |
|---|---|---|
| LA HAYE | 24 Juin 1996 | Hunt, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P04C02)